# EUROPEAN PATENT APPLICATION

(11) **EP 2 837 949 A1**
(43) Date of publication of application: **18.02.2015**
(21) Application number: 14175870.6
(22) Date of filing: 04.07.2014
(51) Int. Cl.: G01S 7/52, A61B 8/00

(54) **Ultrasonic probe, system including the same, and operation method thereof**

(30) Priority: 24.07.2013 KR 20130087613
(71) Applicant: Samsung Electronics Co., Ltd, Gyeonggi-do 443-742 (KR)
(72) Inventor: Kim, Sang-won, Gyeonggi-do (KR); Cho, Jeong, Seoul (KR); Han, Ho-san, Seoul (KR)
(74) Representative: Jacobs, Bart

(57) **Abstract**

Provided are an ultrasonic probe (100), a system including the same, and an operation method thereof. The ultrasonic probe (100) may include a probe head (110) comprising a transducer (210) configured to receive a signal, and a first storage (220) configured to store information, and a probe body (120) comprising an image processor (310) configured to generate an ultrasonic image with the signal received from the probe head (110), wherein the transducer (210) and the image processor (310) are electrically connected to each other by a coupling between the probe head (110) and the probe body (120), and wherein the transducer (210) and the image processor (310) are separated from each other by a separation between the probe head (110) and the probe body (120).

## Description

Apparatuses and methods consistent with exemplary embodiments relate to an ultrasonic probe, a system including the same, and an operation method thereof.

An ultrasonic diagnostic system may irradiate an ultrasonic wave toward an object which may be a body of a person or an animal, detect an echo signal reflected from the body, and display a tomographic image of a tissue in the body by using a monitor thereby provide information which may be used for a diagnosis of the object.

An ultrasonic diagnostic system may include an ultrasonic probe that transmits an ultrasonic wave into an object, and receives an echo signal from the inside of the object. Also, the ultrasonic probe may include a transducer that is disposed inside the ultrasonic probe, and converts between an ultrasonic signal and an electrical signal. The transducer includes an array of a plurality of piezoelectric elements.

In addition to the transducer that converts an electrical signal into an ultrasonic signal, a module that generates an ultrasonic image as an electrical signal corresponding to an echo signal may be built into the ultrasonic probe, thus simplifying the ultrasonic diagnostic system.

However, despite various functions being added to the ultrasonic probe, a kind of image processing module is connected to a kind of transducer, and due to this, it is unable to supply various kinds of ultrasonic images to one ultrasonic probe.

One or more exemplary embodiments include an ultrasonic probe in which a probe head including a transducer is attachable/detachable to/from a probe body including an image processor.

Additional aspects will be set forth in part in the description which follows and, in part, will be apparent from the description, or may be learned by practice of the presented exemplary embodiments.

According to an aspect of an exemplary embodiment, there is provided an ultrasonic probe including a probe head including a transducer configured to receive a signal and a first storage configured to store information, and a probe body including an image processor configured to generate an ultrasonic image with the signal received from the probe head, wherein the transducer and the image processor are electrically connected to each other by a coupling between the probe head and the probe body, and wherein the transducer and the image processor are separated from each other by a separation between the probe head and the probe body.

The probe body may be configured to read identification information from the probe head to authenticate the probe head, wherein the identification information is stored in the first storage unit.

The identification information may include at least one of a serial information, a manufacturing information, and a type information of the probe head.

The identification information may include a combination of at least some of a serial information, a manufacturing information, and a type information of the probe head.

The information may include information about the ultrasonic image that is generated with the signal received by the transducer.

The information may be uploaded from an external device.

The image processor may generate the ultrasonic image on a basis of the information.

The ultrasonic probe may be portable.

The probe body may further include a wireless communicator that wirelessly transmits the ultrasonic image to an external device.

The external device may be a display device.

The coupling may be achieved by inserting a portion of the probe head into the probe body.

According to another aspect of an exemplary embodiment, there is provided an ultrasonic diagnostic system including an ultrasonic probe including a probe head including a transducer configured to receive a signal and a first storage configured to store information, and a probe body including an image processor configured to generate an ultrasonic image with the signal received from the probe head, wherein the transducer and the image processor are electrically connected to each other by a coupling between the probe head and the probe body, and wherein the transducer and the image processor are separated from each other by a separation between the probe head and the probe body, and a display device that wirelessly receives the ultrasonic image from the ultrasonic probe, and displays the ultrasonic image.

The ultrasonic probe generates the ultrasonic image according to a user command received from the display device.

According to another aspect of an exemplary embodiment, there is provided an ultrasonic probe head including a transducer at a front end, a storage that stores identification information of the transducer and information about an ultrasonic image that is generated with a signal output from the transducer, and a connector at a back end.

The information about the ultrasonic image may be uploaded from an external device.

According to another aspect of an exemplary embodiment, there is provided a method of operating an ultrasonic probe, the method including coupling a probe head including a transducer configured to receive a signal to a probe body configured to generate an ultrasonic image, authenticating, using the probe body, the probe head, and generating, using the probe body, the ultrasonic image with the signal received from the probe head when the probe head is successfully authenticated.

The authenticating of the probe head may include authenticating the probe head by using identification information of the probe head stored in the probe head.

The identification information may include at least one of a serial information, a manufacturing information, and a type information of the probe head.

The method may further include wirelessly transmitting the ultrasonic image to an external device.

The external device may be a display device that displays the ultrasonic image.

These and/or other aspects will become apparent and more readily appreciated from the following description of exemplary embodiments, taken in conjunction with the accompanying drawings of which:
FIG. 1 is a diagram schematically illustrating an ultrasonic probe according to an exemplary embodiment;
FIG. 2 is a diagram schematically illustrating an ultrasonic probe according to another exemplary embodiment;
FIGs. 3A and 3B are diagrams schematically illustrating a connection relationship between a first connector of a probe head and a second connector of a probe body according to one or more exemplary embodiments;
FIG. 4 is a diagram schematically illustrating an ultrasonic probe according to another exemplary embodiment;
FIG. 5 is a block diagram schematically illustrating a probe head according to another exemplary embodiment;
FIG. 6 is a block diagram schematically illustrating a probe body according to another exemplary embodiment;
FIG. 7 is a block diagram schematically illustrating a probe body according to another exemplary embodiment;
FIG. 8 is a diagram illustrating an ultrasonic diagnostic system including the ultrasonic probe; and
FIG. 9 is a flowchart illustrating an operation method of an ultrasonic probe according to an exemplary embodiment.

Reference will now be made in detail to exemplary embodiments, examples of which are illustrated in the accompanying drawings, wherein like reference numerals refer to the like elements throughout. In this regard, the present exemplary embodiments may have different forms and should not be construed as being limited to the descriptions set forth herein. Accordingly, the exemplary embodiments are merely described below, by referring to the figures, to explain aspects of the present description.

Hereinafter, exemplary embodiments will be described in detail with reference to the accompanying drawings. Like reference numerals in the drawings denote like elements, and thus descriptions thereof will not be repeated.

The term "object" used herein may include a person, an animal, a part of the person, or a part of the animal. For example, an object may include an organ such as a liver, a heart, a womb, a brain, breasts, an abdomen, or the like, or a blood vessel. Moreover, the term "user" used herein is a medical expert, and may be a doctor, a nurse, a medical technologist, a medical image expert, or the like, or may be an engineer who repairs a medical apparatus. However, the user is not limited thereto.

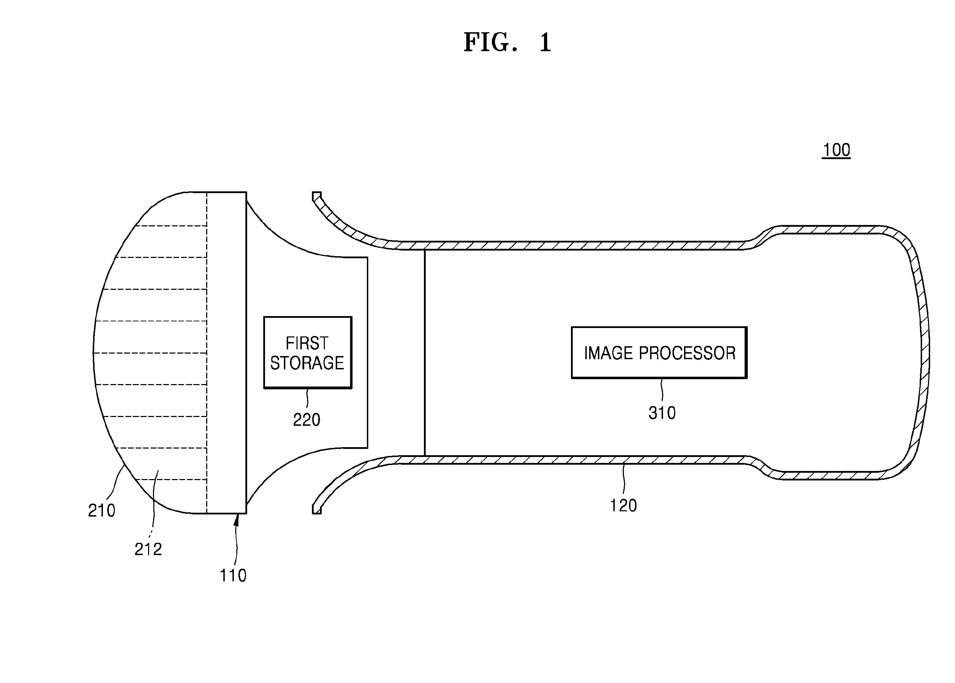

FIG. 1 is a diagram schematically illustrating an ultrasonic probe 100 according to an exemplary embodiment. Referring to FIG. 1, the ultrasonic probe 100 includes a probe head 110 into which a transducer 210 and a first storage 220 storing information are built; and a probe body 120 that includes an image processor 310 that generates an ultrasonic image with a signal received from the probe head 110. The ultrasonic probe 100 is a portable probe that enables a user to move from place to place with the portable device.

The probe head 110 may include a transducer 210 that transmits an ultrasonic wave toward an object, and receives an echo signal of the ultrasonic wave reflected from the object. The transducer 210 may include a plurality of piezoelectric elements 212 that convert between an electrical signal and an acoustic signal. Each of the plurality of piezoelectric elements 212 may be formed by dividing an piezoelectric material into a plurality of materials. For example, each piezoelectric element 212 may be manufactured by dicing a long piezoelectric element. However, the exemplary embodiments are not limited to a method of division-manufacturing the plurality of piezoelectric elements 212. For another example, the plurality of piezoelectric elements 212 may be manufactured by various methods in addition to a method that presses a piezoelectric material with a metal mold to form the plurality of piezoelectric elements 212. The piezoelectric material may be a piezoelectric ceramic which may cause a piezo phenomenon, a single crystalline, or a complex piezoelectric material, and may be generated by combining the material with a polymer.

A plurality of piezoelectric elements that are one-dimensionally arrayed on a plane vertical to a propagation direction of an ultrasonic wave may be called a one-dimensional (1 D) piezoelectric element array. The 1D piezoelectric element array may be a linear array or a curved array. An array type may be variously set depending on a designer's intention. The 1D piezoelectric element array may be easily manufactured and thus may be low in cost. However, it may be difficult for the 1D piezoelectric element array to realize a three-dimensional (3D) stereoscopic image.

A plurality of piezoelectric elements that are two-dimensionally arrayed on a plane vertical to an ultrasonic wave traveling direction may be called a two-dimensional (2D) piezoelectric element array. The 2D piezoelectric element array may be a linear array or a curved array. An array type may be variously set depending on a designer's intention. Here, a 2D piezoelectric element unit appropriately delays an input time of each signal respectively input to a plurality of piezoelectric elements, and transmits the delayed signals toward an object along an external scan line through which an ultrasonic wave is transmitted. Therefore, the 2D piezoelectric element unit obtains a stereoscopic image by using a plurality of echo signals.

The transducer 210 is an element which converts between an ultrasonic signal and an electrical signal, but the transducer 210 is not limited thereto. In addition, the transducer 210 may be implemented as a capacitive micromachined ultrasonic transducer (cMUT) that converts between an ultrasonic signal and an electrical signal according to a capacitance change, a magnetic micromachined ultrasonic transducer (mMUT) that converts between an ultrasonic signal and an electrical signal according to an magnetic-field change, or an optical ultrasonic detector that converts between an ultrasonic signal and an electrical signal according to an optical-characteristic change.

The probe head 110 may include the first storage 220 that stores various information. The first storage 220 may store identification information of the probe head 110 or information about a generable ultrasonic image. Here, the identification information may include at least one of serial information, manufacturing information, and type information regarding the probe head 110. The type information may include operating frequency information of a piezoelectric element, information about whether a piezoelectric element array is a 1D array or a 2D array, and information about whether the piezoelectric element array is a linear array or a curved array. The identification information may be generated by combining at least two of the serial information, the manufacturing information, and the type information, or may be generated by combining at least two of some of the serial information, some of the manufacturing information, and some of the type information.

Information regarding an ultrasonic image denotes information about a kind of ultrasonic image that may be generated by using a signal received from the transducer 210. The kind of ultrasonic image may be divided into a brightness (B) mode image in which a level of an ultrasonic echo signal reflected from an object is expressed as brightness, a Doppler mode image in which an image of a moving object is expressed as a spectrum type by using the Doppler effect, a motion (M) mode image that shows a motion of an object with time at a certain place, an elastic mode image in which a reaction difference between when compression is applied to an object and when compression is not applied to the object is expressed as an image, and a color (C) mode image in which a speed of a moving object is expressed as a color by using the Doppler effect. Furthermore, the kind of ultrasonic image may be divided into mode-dimensional images such as a 1D image, a 2D image, a three-dimensional (3D) image, and a fourth-dimensional (4D) image.

Moreover, the first storage 220 may store a program that generates a generable ultrasonic image. Information about an ultrasonic image and the program that generates the ultrasonic image may be uploaded by an external device. Here, the external device may be a server that stores information about an ultrasonic image and various programs that generate the ultrasonic image.

The first storage 220 may include at least one type of storage medium such as a flash memory, a hard disk, a multimedia micro card, a card type memory (a secure digital (SD) card, an extreme digital (XD) card, or the like), a random access memory (RAM), a static random access memory (SRAM), a read-only memory (ROM), an electrically erasable programmable read-only memory (EEPROM), and a programmable read-only memory (PROM).

The probe body 120 may include the image processor 310 that generates an ultrasonic image with a signal received from the probe head 110, namely, the transducer 210. The image processor 310 may receive the signal received from the transducer 210, and process the received signal by using a program corresponding to a kind of ultrasonic image.

For example, when information about an ultrasonic image indicates the B mode image, the image processor 310 may perform a filtering operation on the signal received from the probe head 110 to remove a noise component, and perform a scan conversion operation to generate a B-mode ultrasonic image including a plurality of scan lines.

Alternatively, when information about an ultrasonic image is a Doppler image, the image processor 310 may adjust a grain of the signal received from the probe head 110 on the basis of a predetermined color gain, and perform frequency analysis for acquiring information associated with a motion of an object from the adjusted signal to generate Doppler data. Furthermore, the image processor 310 may generate a Doppler image indicating a motion of the object on the basis of the Doppler data. The Doppler image expresses information about an average speed, variance, and power component of a moving object, and may be displayed with a color (a color Doppler image) or a frequency spectrum (a spectrum Doppler image).

Such a Doppler image may include a Doppler image about a continuous image such as a moving image, in addition to a Doppler image about a still image, or may include both a Doppler image (a 2D Doppler image) about a plane space and a Doppler image (a 3D Doppler image) about a stereoscopic space. Also, the Doppler image may include a blood flow Doppler image (also called a color Doppler image) indicating a flow of blood and a tissue Doppler image indicating a motion of a tissue.

Moreover, when information about an ultrasonic image indicates a 3D image, the image processor 310 may generate volume data with the signal received from the probe head 110, and perform volume rendering on the volume data to generate a 3D ultrasonic image.

The volume rendering is technology that generates a 2D projection image about a 3D discretely sampled data set such as the volume data. For example, the image processor 310 may perform a volume rendering operation by using a ray casting method that casts virtual rays onto an object located in a virtual space to calculate reflective light.

The probe head 110 is attachable/detachable to/from the probe body 120. Furthermore, the transducer 210 and the image processor 310 may be electrically connected to each other by a coupling between the probe head 110 and the probe body 120, and the transducer 210 and the image processor 120 may be separated from each other by separation between the probe head 110 and the probe body 120. For example, the coupling between the probe head 110 and the probe body 120 may be made by inserting a portion of the probe head 110 into the probe body 120. Here, the portion of the probe head 110 may be an area in which the transducer 210 is not disposed. For example, the portion of the probe head 110 may be an area opposite to an area of the probe head 110 in which the transducer 210 is disposed.

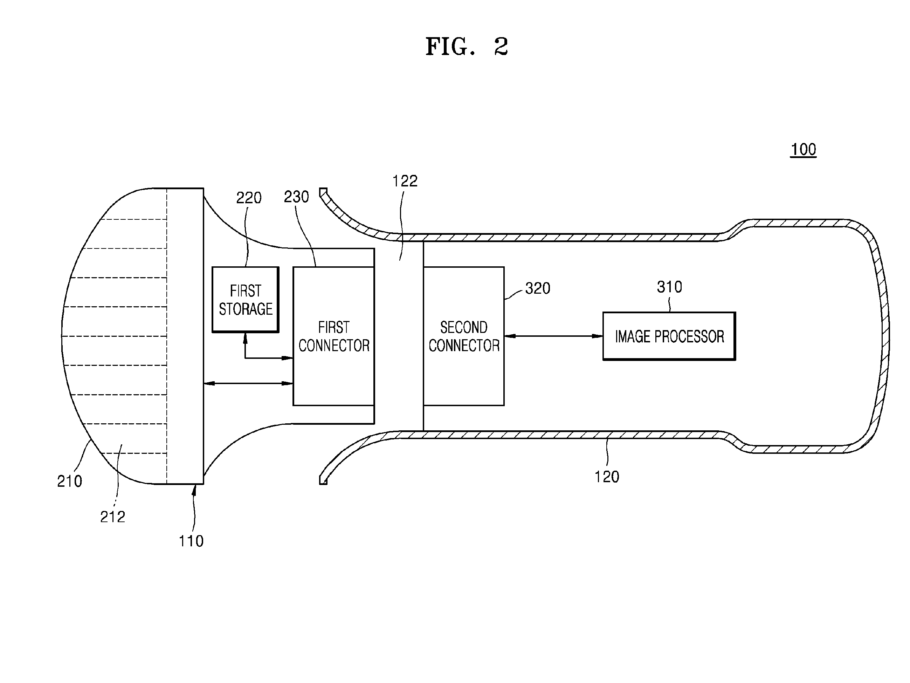

For an electrical connection between the probe head 110 and the probe body 120, the probe head 110 may include a first connector, and the probe body 120 may include a second connector. FIG. 2 is a diagram schematically illustrating an ultrasonic probe 100 according to another exemplary embodiment. The probe head 110 of FIG. 2 may include a first connector 230 that may transfer an electrical signal of the transducer 210 to the probe body 120. The first connector 230 may be electrically connected to the first storage 220, and thus, the probe body 120 may read information from the first storage 220 through the first connector 230. The first connector 230 may be disposed in an area opposite to an area of the probe head 110 in which the transducer 210 is disposed, and may be partially exposed. Therefore, when the probe head 110 is coupled to the probe body 120, the first connector 230 is electrically connected to a second connector 320 of the probe body 120. Hereinafter, an area with the transducer 210 disposed therein is referred to as a front end of the probe head 110, and an area in which the first connector 230 of the probe head 110 is exposed is referred to as a rear end of the probe head 110.

Moreover, the probe body 120 may include the second connector 320 that transfers a signal, received from the probe head 110, to the image processor 310. The second connector 320 is disposed in an opening 122 of the probe body 120, and is partially exposed. Therefore, when the probe head 110 is coupled to the probe body 120, the second connector 320 is electrically connected to the first connector 230. Hereinafter, an area in which the second connector 320 is exposed is referred to as a front end of the probe body 120. The first and second connectors 230 and 320 may be formed of a conductive material.

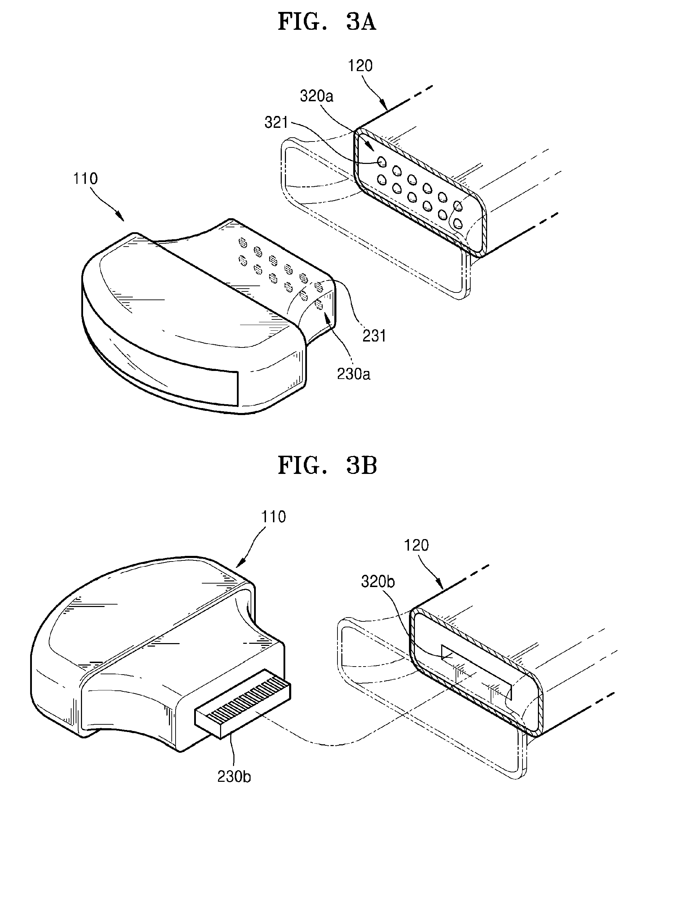

FIGS. 3A and 3B are diagrams schematically illustrating a connection relationship between the first connector of the probe head 110 and the second connector of the probe body 120 according to one or more exemplary embodiments.

As illustrated in FIG. 3A, a first connector 230a may include a plurality of protrusion portions 231. The plurality of protrusion portions protrude at a rear end of the probe head 110. A second connector 320a may include a plurality of grooves 321. The plurality of grooves 321 may be disposed at a front end of the probe body 120 and in an area corresponding to the respective protrusion portions 231. The protrusion portions 231 may be formed of a conductive material, and the plurality of grooves 321 may be surrounded by a conductive material. Therefore, each of the plurality of protrusion portions is inserted into a corresponding groove 321, and thus, the first connector 230a is connected to the second connector 320a, and the probe head 110 is electrically connected to the probe body 120.

Alternatively, as illustrated in FIG. 3B, the first connector 230b may be a plug type, and the second connector 320b may be a hook type. Therefore, when the first connector 230b is connected to the second connector 320b, the probe head 110 is electrically connected to the probe body 120.

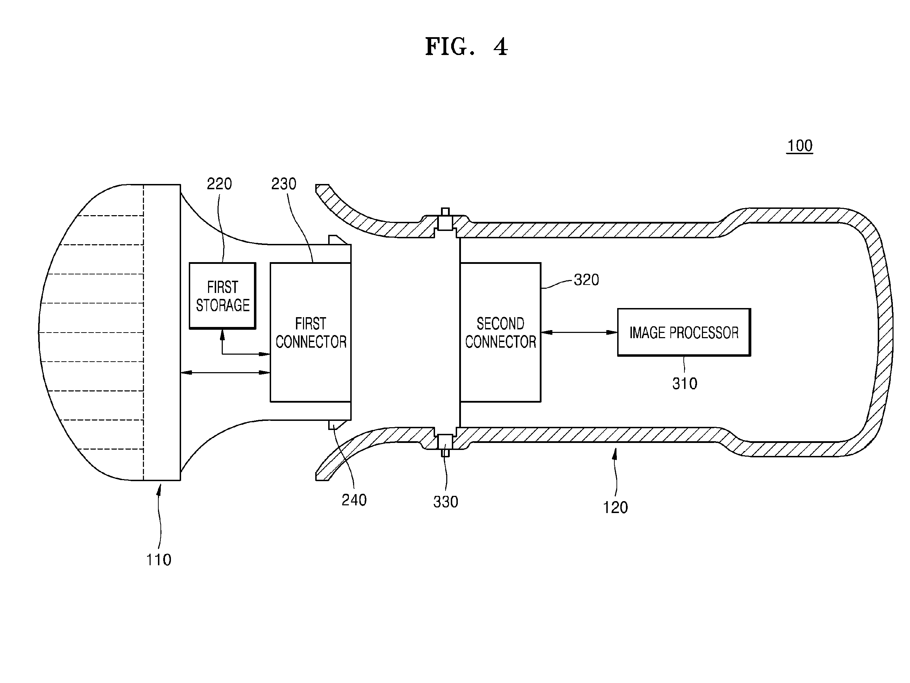

FIG. 4 is a diagram schematically illustrating an ultrasonic probe according to another exemplary embodiment. In comparison with FIG. 2, a first coupler 240 is disposed at a side portion of the probe head 110, and a second coupler 330 is disposed at a side portion of the probe body 120. The first coupler 240 may be formed as a hook type to protrude at the side portion of the probe head 110. Also, the first coupler 240 may be formed of an elastic material, and has an elastic restoring force in an outer direction. Furthermore, the second coupler 330 may also be formed similar to the first coupler 240. The inside of the second coupler 330 may be empty such that the first coupler 240 is disposed. Therefore, when a user inserts the rear end of the probe head 110 into the opening 122 of the probe body 120, the first coupler 240 may protrude into an area of the second coupler 330 disposed therein, and thus, the probe head 110 may be coupled to the probe body 120. In addition, when the probe head 110 is coupled to the probe body 120, the user may disconnect the probe head 110 from the probe body 120 by, form the outside the probe body 120, pushing the second coupler 330, thereby separating the probe head 110 from the probe body 120.

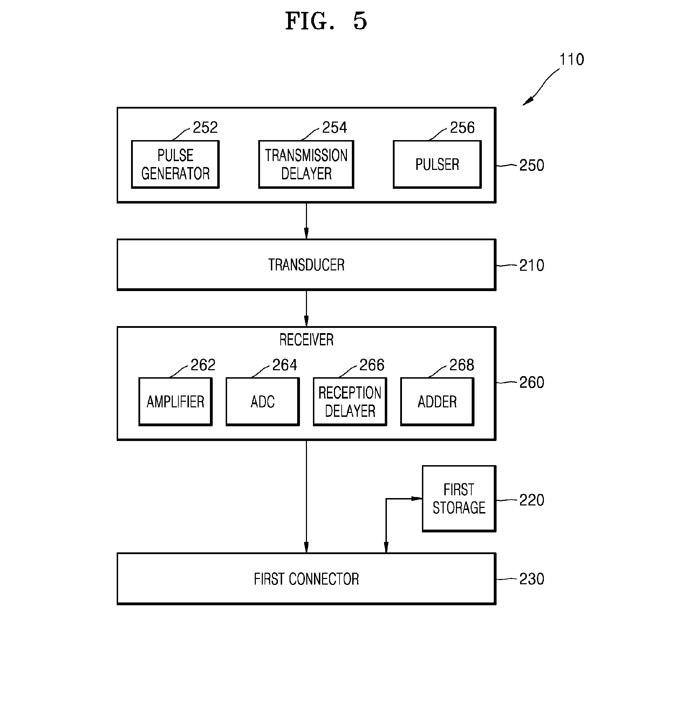

FIG. 5 is a block diagram schematically illustrating a probe head 110 according to another exemplary embodiment. The probe head 110 of FIG. 5 may include a transmitter 250 that transmits an electrical signal to the transducer 210 and a receiver 260 that receives an electrical signal corresponding to an echo signal.

The transmitter 250 supplies a driving signal to the transducer 210. The transmitter 250 may include a pulse generator 252, a transmission delayer 254, and a pulser 256.

The pulse generator 252 generates a rate pulse for generating a transmission ultrasonic wave based on a pulse repetition frequency (PRF). The transmission delayer 254 applies a delay time, used to decide transmission directionality, to the rate pulse generated by the pulse generator 252. A plurality of the rate pulses with the delay time applied thereto correspond to a plurality of piezoelectric vibrators included in the transducer 210, respectively. The pulser 256 applies a driving signal (or a driving pulse) to the transducer 210 at a timing which corresponds to each of the plurality of rate pulses with the delay time applied thereto.

The receiver 260 processes a reflected signal that originated from the transducer 210 to generate ultrasonic data, and may include an amplifier 262, an analog-to-digital converter (ADC) 264, a reception delayer 266, and an adder 268.

The amplifier 262 amplifies the signal received from the transducer 240, and the ADC 264 analog-to-digital converts the amplified signal. The reception delayer 266 applies a delay time, used to decide reception directionality, to the digital-converted signal. The adder 268 adds signals processed by the reception delayer 266 to generate ultrasonic data. A reflection component from a direction, decided based on the reception directionality, may be emphasized by an addition processing performed by the adder 268.

In FIG. 5, the transmitter 250 and the receiver 260 have been described above as being included in the probe head 110, but are not limited thereto. At least one of the transmitter 250 and the receiver 260 may be included in the probe body 120.

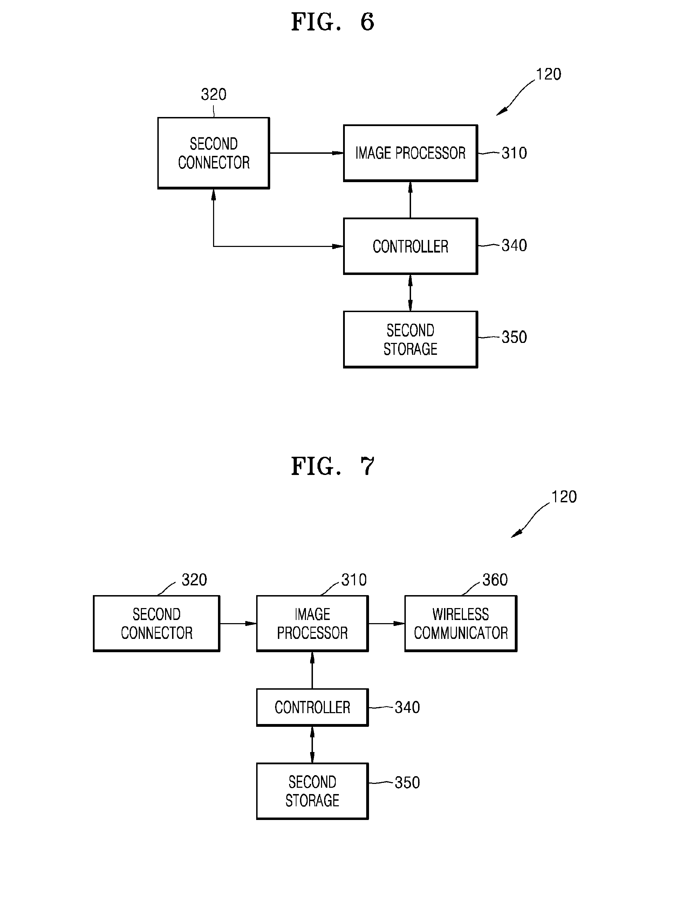

FIG. 6 is a block diagram schematically illustrating a probe body 120 according to another exemplary embodiment. The probe body 120 of FIG. 6 may further include a controller 340 that authenticates the probe head 110 and a second storage 350 that stores identification information of the probe head 110 enabling control. Specifically, when the probe head 110 is electrically connected to the probe body 120 by coupling the probe head 110 to the probe body 120, the controller 340 may read the identification information (stored in the first storage 220 of the probe head 110) on the probe head 110 to authenticate the probe head 110. When the read identification information matches the identification information (stored in the first storage 220) on the probe head 110 enabling control, the controller 340 determines the connected probe head 110 as a controllable device. However, when the read identification information does not match the identification information stored in the first storage 220, the controller 340 determines the connected probe head 110 as an uncontrollable device.

Moreover, the controller 340 may control the image processor 310 so as to generate an ultrasonic image on the basis of information about an ultrasonic image which is stored in the probe head 110. For example, when the ultrasonic image is the B mode image, the controller 340 may control the image processor 310 so as to generate the B mode image.

The second storage 350 may store various information obtained through processing using the ultrasonic probe 100. For example, the second storage 350 may store identification information of the probe head 110 enabling control, an algorithm to generate each ultrasonic image, and a program for a user interface.

FIG. 7 is a block diagram schematically illustrating a probe body 120 according to another exemplary embodiment. The probe body 120 of FIG. 7 may include a wireless communicator 360 communicable with an external device. Therefore, the wireless communicator 360 may perform data communication according to the digital imaging and communications in medicine (DICOM) standard.

The wireless communicator 360 may transmit and receive an ultrasonic image of an object to and from an external device over a network. In particular, the wireless communicator 360 may transmit an ultrasonic image to a display device 12 (an external device) in wireless communication with the display device 12 such that the display device 12 displays the ultrasonic image. In addition, the wireless communicator 360 may perform data communication with a portable terminal of a doctor or a patient, in addition to a server or medical apparatus of a hospital.

The wireless communicator 360 may be wirelessly connected to a network, and may exchange data with a server, a medical apparatus, or a portable terminal. For example, the wireless communicator 360 may receive and store identification information of the probe head 110 enabling control, an algorithm to generate each ultrasonic image, and a program for a user interface from any one of the server, the medical apparatus, or the portable terminal. The wireless communicator 360 may include one or more elements that enable communication with an external device, and for example, include a short-distance communication module, a mobile communication module, etc.

The short-distance communication module denotes a module for short-distance communication within a certain distance. Short-distance communication technology, according to an exemplary embodiment, may include wireless LAN, Wi-Fi, Bluetooth, Zigbee, Wi-Fi direct (WFD), ultra wideband (UWB), infrared data association (IrDA), Bluetooth low energy (BLE), and near field communication (NFC), but the short-distance communication technology is not limited thereto. The mobile communication module transmits and receives a radio frequency (RF) signal to and from a base station, an external terminal, and a server over a mobile communication network.

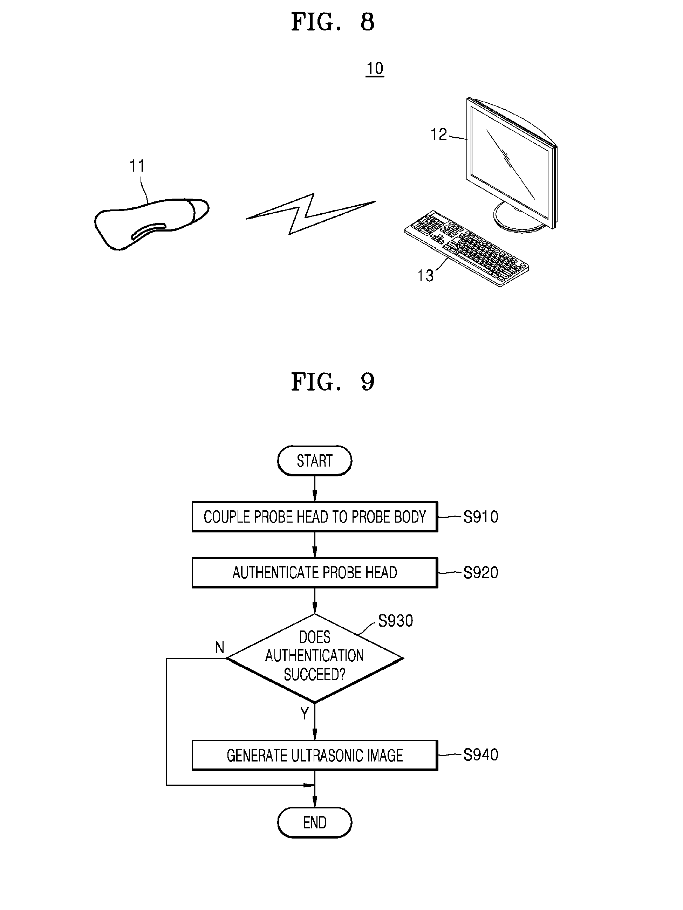

FIG. 8 is a diagram illustrating an ultrasonic diagnostic system 10 including the ultrasonic probe 11.

As illustrated in FIG. 8, the ultrasonic diagnostic system 10 may include an ultrasonic probe 11, which transmits and receives an ultrasonic wave to generate an ultrasonic image, and the display device 12 that displays the ultrasonic image. The ultrasonic probe 11 has been described above, and thus, its detailed description will not be repeated here. The display device 12 may display information obtained through processing by the ultrasonic probe 10. For example, the display device 12 may display an ultrasonic image generated by the image processor 310 of the ultrasonic probe 11, and display a graphics user interface (GUI) for requesting a user's input.

The display device 12 includes one display unit, which may include at least one of a projector, a liquid crystal display (LCD), a thin film transistor-liquid crystal display (TFT-LCD), an organic light-emitting diode (OLED), a flexible display, a 3D display, and an electrophoretic display.

When the display unit and the touch panel form a layer structure and thus are configured with a touch screen, the display unit may be used as a user input unit in addition to an output unit. The touch panel may be implemented as various types such as a contact capacitive type, a press resistive type, an infrared sensing type, a surface ultrasonic conductive type, an integration tension measurement type, and a piezo effect type.

Moreover, the ultrasonic diagnostic system 10 may further include a user input unit 13 that enables a user to input data for controlling the medical apparatus 100. In the drawing, a keypad is illustrated as the user input unit 12, but is not limited thereto. The user input unit 13 may further include various input devices such as a mouse, a touch panel, a jog wheel, a jog switch, a microphone, a camera sensor, etc.

The following description is of an operation method of the ultrasonic probe when the probe head is coupled to the probe body. FIG. 9 is a flowchart illustrating an operation method of the ultrasonic probe according to an exemplary embodiment.

Referring to FIG. 9, in operation S910, the probe head 110 with the built-in transducer 210 is coupled to the probe body 120 that generates an ultrasonic image. For example, by inserting the rear end of the probe head 110 into the probe body 120, the first connector 230 may be connected to the second connector 320. Therefore, the probe head 110 is electrically connected to the probe body 120.

When the probe head 110 is coupled to the probe body 120, the probe body 120 authenticates the probe head 110 in operation S920. For example, the controller 340 of the probe body 120 reads identification information of the probe head 110 from the first storage 220 included in the probe head 110. The controller 340 may authenticate whether the identification information of the probe head 110 matches identification information stored in the second storage 350. When the identification information of the probe head 110 matches the identification information stored in the second storage 350, the controller 340 determines the probe head 110 as being successfully authenticated.

Alternatively, when the probe head 110 is coupled to the probe body 120, the controller 340 may transmit a user interface, stored in the second storage 350, to the display device 12 (an external device) such that the user interface is displayed by the display device 12. When a user command for selecting a probe head 110 is input through the user interface, the controller 340 may authenticate the probe head 110 according to whether the read identification information of the probe head 110 matches identification information of the selected probe head 110. Alternatively, the controller 340 may receive identification information of a specific probe head 110 from an external device, and compare the read identification information of the probe head 110 and the received identification information of the specific probe head 110, thereby authenticating the coupled probe head 110. The identification information may include at least one of serial information, manufacturing information, and type information of the probe head 110.

When the probe head 110 is successfully authenticated in operation S930-Y, the probe body 120 may generate an ultrasonic image with a signal received from the probe head 110 in operation S940. When the authentication of the probe head 110 succeeds, the controller 340 reads information about an ultrasonic image which is stored in the first storage 220. The controller 340 may process the signal received from the probe head 110 on the basis of the information about the ultrasonic image, and generate an ultrasonic image. Different kinds of ultrasonic images may be generated for each transducer 210. The probe header 110 may store information about an ultrasonic image generable through a corresponding transducer 210 and a program used to generate the ultrasonic image, thus reducing a storage capacity of the second storage 350 of the probe body 120. Therefore, a portable ultrasonic probe 100 is implemented. The probe body 120 may wirelessly transmit the generated ultrasonic image to an external device, for example, the display device 12.

In the ultrasonic probe according to the exemplary embodiments, various transducers are adhered to the probe body including the image processor, thus generating various kinds of ultrasonic images.

In the ultrasonic probe according to the exemplary embodiments, information to generate an ultrasonic image is stored in the probe head, thus simplifying a function of the probe body.

According to another exemplary embodiment, a first connector 230 of a probe head 110 may be a Universal Serial Bus (USB) plug. Further, after authentication S930 and ultrasonic image generation S940 the image processor 310 and controller 340 in the probe body 120 may, in addition to transmitting the generated image externally and/or storing it locally in the probe body 120, may also transmit the generated ultrasonic image back to the probe head 110 through the USB plug storing the image on the first storage 220 in a known image format such as a JPEG, a TIFF, a BMP, a PNG, a GIF, etc, or in a known video format. Further, the controller 340 may remove from the first memory 220 any proprietary software and data. Accordingly, once a user completes a use of the imaging ultrasonic probe, the probe head may be disconnected and given to the person who was the object of the imaging and diagnosis.

It should be understood that the exemplary embodiments described herein should be considered in a descriptive sense only and not for purposes of limitation. Descriptions of features or aspects within each exemplary embodiment should typically be considered as available for other similar features or aspects in other exemplary embodiments.

While one or more exemplary embodiments have been described with reference to the figures, it will be understood by those of ordinary skill in the art that various changes in form and details may be made therein without departing from the inventive concept as defined by the following claims.

## Claims

1. An ultrasonic probe comprising:
a probe head comprising a transducer configured to receive a signal and a first storage configured to store information; and
a probe body comprising an image processor configured to generate an ultrasonic image with the signal received from the probe head,
wherein the transducer and the image processor are electrically connected to each other by a coupling between the probe head and the probe body, and
wherein the transducer and the image processor are separated from each other by a separation between the probe head and the probe body.

2. The ultrasonic probe of claim 1, wherein the probe body is configured to read identification information from the probe head to authenticate the probe head, wherein the identification information is stored in the first storage unit.

3. The ultrasonic probe of claim 2, wherein the identification information includes at least one of a serial information, a manufacturing information, and a type information of the probe head or a combination of at least some of a serial information, a manufacturing information, and a type information of the probe head.

4. The ultrasonic probe of any one of claims 1 to 3, wherein the information includes information about the ultrasonic image that is generated with the signal received by the transducer.

5. The ultrasonic probe of claim 4, wherein the information is uploaded from an external device.

6. The ultrasonic probe of claim 4, wherein the image processor generates the ultrasonic image on a basis of the information.

7. The ultrasonic probe of any one of claims 1 to 6, wherein the ultrasonic probe is portable.

8. The ultrasonic probe of any one of claims 1 to 7, wherein the probe body further includes a wireless communicator that wirelessly transmits the ultrasonic image to an external device.

9. The ultrasonic probe of any one of claims 1 to 8, wherein the coupling is achieved by inserting a portion of the probe head into the probe body.

10. An ultrasonic diagnostic system comprising:
an ultrasonic probe comprising:
a probe head comprising a transducer configured to receive a signal and a first storage configured to store information; and
a probe body comprising an image processor configured to generate an ultrasonic image with the signal received from the probe head,
wherein the transducer and the image processor are electrically connected to each other by a coupling between the probe head and the probe body, and
wherein the transducer and the image processor are separated from each other by a separation between the probe head and the probe body; and
a display device that wirelessly receives the ultrasonic image from the ultrasonic probe, and
displays the ultrasonic image.

11. The ultrasonic diagnostic system of claim 10, wherein the ultrasonic probe generates the ultrasonic image according to a user command received from the display device.

12. A method of operating an ultrasonic probe, the method comprising:
coupling a probe head comprising a transducer configured to receive a signal to a probe body configured to generate an ultrasonic image;
authenticating, using the probe body, the probe head; and
generating, using the probe body, the ultrasonic image with the signal received from the probe head when the probe head is successfully authenticated.

13. The method of claim 12, wherein the authenticating of the probe head includes authenticating the probe head by using identification information of the probe head stored in the probe head.

14. The method of claim 13, wherein the identification information includes at least one of a serial information, a manufacturing information, and a type information of the probe head.

15. The method of any one of claims 12 to 14, further comprising: wirelessly transmitting the ultrasonic image to an external device.
